**Europäisches Patentamt**

**European Patent Office**

**Office européen des brevets**

(11) Publication number: **0 143 208**
**B1**

(12) **EUROPEAN PATENT SPECIFICATION**

(45) Date of publication of patent specification: **04.11.87**

(21) Application number: **84110390.6**

(22) Date of filing: **31.08.84**

(51) Int. Cl.⁴: **A 61 M 16/00**

(54) Squeeze bag.

(30) Priority: **31.08.83 DK 3967/83**

(43) Date of publication of application:
**05.06.85 Bulletin 85/23**

(45) Publication of the grant of the patent:
**04.11.87 Bulletin 87/45**

(84) Designated Contracting States:
**DE FR GB IT SE**

(56) References cited:
**CH-A- 552 988**
**GB-A-1 381 951**
**US-A-3 009 459**
**US-E- 28 486**

(73) Proprietor: **TESTA-LABORATORIUM A/S**
**Sondre Ringvej 49**
**DK-2600 Glostrup (DK)**

(72) Inventor: **Grane, Christian**
**Bronsholmdalsvej 47**
**DK-2980 Kokkedal (DK)**

(74) Representative: **Patentanwälte Leinweber &**
**Zimmermann**
**Rosental 7/II Aufg.**
**D-8000 München 2 (DE)**

Courier Press, Leamington Spa, England.

### Description

This invention relates to a squeeze bag for a manually operated resuscitator, said squeeze bag comprising an outer airtight and flexible bladder, having a gas outlet and a gas inlet, a self-expanding foldable insert provided within said bladder, the gas outlet having inserted therein coupling means for coupling the bladder to a valve system of the resuscitator and the gas inlet having inserted therein a gas inlet valve.

The specifications of US patents Nos. 3.009.456 and Re. 28.486 disclose squeeze bags of the above mentioned type. These prior art squeeze bags suffer from the drawback that it is difficult or even impossible to remove the insert from the bladder and consequently the bladder cannot be effectively sterilized.

An attempt to facilitate the removal of the insert from the bladder by increasing the size of the gas inlet or gas outlet has not been successful because of the problems created in providing an effective seal between the bladder and/or the insert on one hand and the coupling means or the inlet valve on the other.

The object of the invention is to provide a squeeze bag of the above mentioned type which allows an easy removal and reinsertion of the insert and which, when assembled, is effectively sealed relative to the surroundings.

This object is achieved by the squeeze bag of the invention which is characterized in that the gas outlet is sealed to a sealing ring which surrounds and fits tightly to the coupling means which is in the form of a pipe section, said coupling means being detachable from said sealing ring and being connected with the insert, and that the gas inlet is such that the inlet valve may be removed and that subsequent to which the insert and the pipe section attached thereto can be removed through said gas inlet with the insert in folded state.

When removing the insert from the bladder, the inlet valve is initially removed from the gas inlet of the bladder. Subsequently, the insert is folded. This may be effected by placing one edge of the insert parallel with the opposite edge and by further folding the edges of the body thus obtained. The folded insert is then pulled out through the gas inlet of the bladder, and at the same time the pipe section is pulled or pushed out of engagement with the sealing ring so as to obtain a complete separation of the two parts.

When the squeeze bag is to be assembled, the insert is folded as described above and the folded insert and the pipe section with the free end of the latter pointing forward are inserted in the bladder. If the pipe section has a proper length, it is easy to introduce the free end thereof in the hole of the sealing ring. Both the removal of the insert from the squeeze bag as well as the assembling thereof can be effected in less than one minute.

The pipe section preferably has a slightly conical exterior shape, e.g. with a conicity of from 1:10 to 1:40 and with the diameter decreasing in the direction towards its free end. Such a shape facilitates both the removal and the insertion of the pipe section in the sealing ring and also effectively seals one part to the other. The pipe section is preferably attached to the insert by stretching the edge of a hole in the insert wall and by placing the stretched edge in an exterior annular groove on the exterior surface of the pipe section.

The exterior surface of the pipe section preferably also comprises an annular bead having an outer diameter which is slightly higher than the internal diameter of the sealing ring. This bead prevents the pipe section from moving into the bladder during use and thus prevents leakage between the pipe section and the bladder.

The sealing ring provided at the gas outlet of the bladder performs two functions, viz. to maintain the bladder in position during the removal of the insert and to provide an effective seal between the pipe section and the bladder.

The sealing ring may be moulded and may be an integral part of the bladder, but in a preferred embodiment of the invention it is composed of an inner ring and an outer locking ring and the edge of the outlet of the bladder is fixed between these two rings. The outer locking ring which preferably is elastic may for example consist of rubber, whereas the inner ring is rigid and consists of e.g. a hard resin or a metal. However, the outer locking ring may also be rigid and the inner ring may be elastic.

The bladder preferably consists of rubber and in that case it may have a wall thickness of about 0.7 mm.

As mentioned above the insert is self-expanding but at the same time foldable. It preferably consists of rubber and has a wall thickness which is greater than that of the bladder.

The insert preferably comprises holes provided in the walls thereof. Thus, the presence of air in the space between the bladder and the insert enables the user to ascertain the pressure within the squeeze bag or bladder through his fingers. Additionally, the presence of such holes in the insert walls makes the operation of the bag less tiring when used over extended periods.

The air inlet valve is a one-way valve which opens when the pressure within the bladder becomes subatmospheric and which closes when the pressure becomes superatmospheric. The valve (except for the valve flap) is preferably composed of a rigid material, e.g. metal or hard resin, so that the valve cannot be deformed when an airtight connection is provided between the bladder and the valve. In a preferred embodiment of the squeeze bag of the invention the inlet valve comprises an internal annular flange and an external compression nut cooperating with said annular flange in such a manner that the edge of the inlet of the bladder may be fixed between the internal annular flange and the compression nut. This embodiment permits an easy removal of the inlet valve from the gas inlet of the bladder when the insert and the pipe section are to be removed therefrom and also makes it possible to provide an effective seal between the bladder and optionally also the insert on one hand and the valve on the other hand.

In order to reduce the friction when the compression nut is pressed against the edge of the gas inlet of the bladder, an antifriction ring is preferably provided between the compression nut and the edge of the gas inlet of the bladder.

The invention will now be described in further detail with reference to the drawings, in which

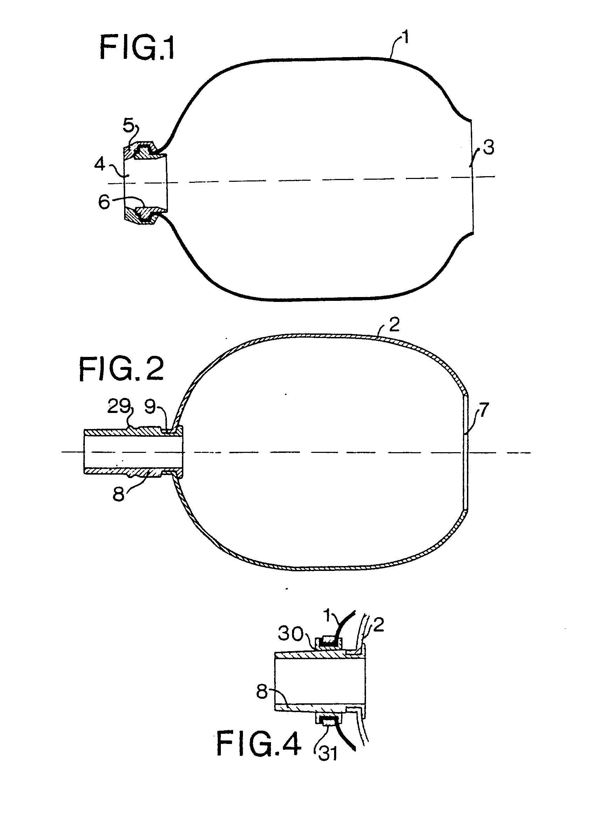

Fig. 1 shows a sectional view of a bladder for use in a squeeze bag according to the invention,

Fig. 2 shows a sectional view of an insert for use in a squeeze bag according to the invention,

Fig. 3 shows an enlarged sectional view of a preferred embodiment of the squeeze bag according to the invention, said squeeze bag comprising the bladder illustrated in Fig. 1 and the insert illustrated in Fig. 2, said bag being connected to the valve system of a resuscitator, and

Fig. 4 shows a sectional view of a pipe section and the parts attached thereto in another embodiment of the squeeze bag of the invention.

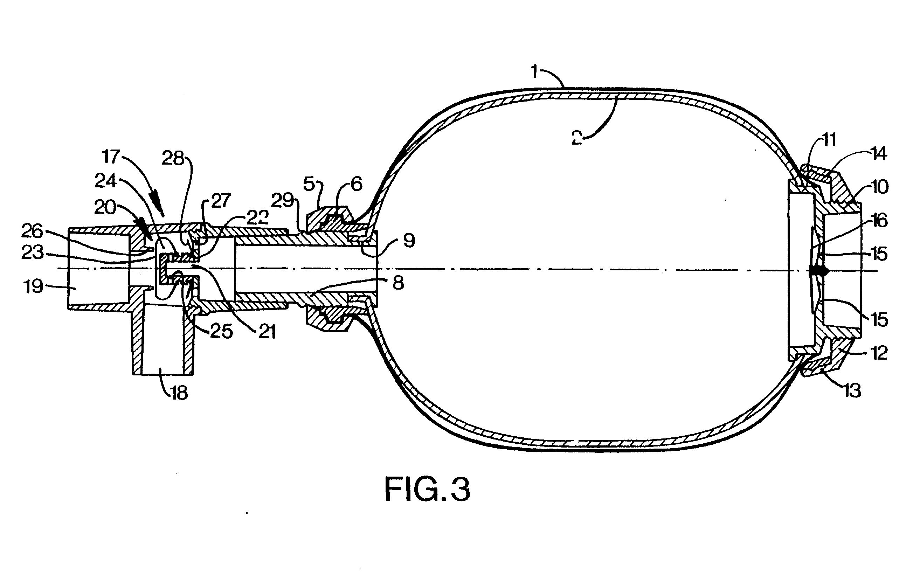

The squeeze bag illustrated in Fig. 3 comprises an elongated rubber bladder 1 and a self-expanding foldable elongated insert 2 provided therein. A gas inlet 3 is provided at one end of the bladder and a gas outlet 4 of the opposite end. The edge of the gas outlet 4 is sealed to a sealing ring consisting of an outer elastic ring 5 preferably consisting of rubber and an inner rigid ring 6 preferably consisting of hard resin.

An opening 7 of essentially the same size and shape as the gas inlet 3 of the bladder 1 is provided at one end of the insert 2 and the opposite end of said insert comprises a further opening having inserted therein a rigid pipe section 8, preferably consisting of hard resin. The external surface of the pipe section 8 comprises an annular groove 9 incorporating the edge of the opening in the insert. The exterior surface of the pipe section 8 further comprises an annular bead 29.

In the assembled squeeze bag, cf. Fig. 3, the gas inlet 3 of the bladder 1 and the opening 7 of the insert 2 are closed by an inlet valve consisting of an inner valve body 10 having an internal annular flange 11 and a compression nut 12. The compression nut 12 comprises an annular flange 13 and an internal thread corresponding to an external thread on the inner valve body 10.

The flanges 11 and 13 are of such a shape that the edges of the openings 3 and 7 can be pressed together so as to provide an airtight connection between the bladder 1 and the insert 2. In order to facilitate the fastening of the compression nut 12 against the valve body there is provided an antifriction ring 14 between the flange 13 and the edge of the opening 3 of the bladder 1.

Around the central portion of the inner valve body 10 there are provided passages 15 which on the inside of the valve are closed by a valve flap 16 attached centrally to the valve body 10. The valve flap 16 has such a shape that it closes the passages 15 when the pressure within the bladder becomes superatmospheric and allows air to flow into the bladder when the pressure becomes subatmospheric.

When the squeeze bag has been assembled the pipe section 8 forms an airtight connection with the sealing ring and the annular bead 29 serves to maintain this state.

The valve system illustrated in Fig. 3 comprises a valve casing 17 comprising a pipe joint 18 capable of being attached to a breathing mask (not shown) and an exhalation pipe 19 as well as a double-seat valve 20 screwed into the valve casing 17. The double-seat valve 20 comprises a central passage 21 surrounded by an annular body 22. The annular body 22 is an airtight connection with the edge of a bellow 23 surrounding a zone 24 which through passages 25 is connected with the central passage 21. The bellow 23 is of such a shape that when the pressure within the bladder is increased to superatmospheric pressure, the bellow is pressed against an annular valve seat 26 in the central portion of the valve casing 17.

The double-seat valve 20 comprises passages 27 provided in a zone surrounding the central passage of said double-seat valve. The passages 27 connect the interior of the bladder 1 with the pipe joint 18 and also cooperate with an annular valve flap 28 which closes the passages 27 when the pressure within the pipe joint 18 exceeds the pressure within the bladder 1.

The operation of the squeeze bag shown is as follows:

When the bladder 1 and the insert 2 are manually compressed, the pressure is increased within the interior of the bladder 1. This increased pressure causes the valve flap 16 to close the passages 15. At the same time the bellow 23 is pressed against the valve seat 26 so as to disrupt a connection, if any, between the pipe joint 18 and the exhalation passage 19. Therefore, air flows from the interior of the bladder 1 through the passages 27 into the passage 18 and further into the air passages of the patient. When the pressure on the bladder 1 is released, it will attempt to attain its original shape under the action of the self-expanding insert 2, and this causes a subatmospheric pressure to be created therein. As a result thereof the passages 27 will be closed by the valve flap 28, and the bellow 23 is pulled away from the valve seat 26 so as to connect the air passages of the patient with the surrounding atmosphere.

Consequently, an exhalation may take place. At the same time the passages 15 are opened, thus permitting fresh air to flow into the bladder 1 which then becomes ready for renewed artificial respiration.

When the squeeze bag has to be dismantled for sterilization, the valve system is initially removed from the pipe section 8 and the compression nut 12 is removed so as to allow the inlet valve to be detached. Subsequently, the insert 2 is folded and is removed from the bladder 1 together with the pipe section 8 which previously has been pushed out of engagement with the ring 5 through the gas outlet 3 of the bladder 1.

When the squeeze bag has to be assembled, the above mentioned steps are carried out in reverse order.

The embodiment of the squeeze bag according to the invention illustrated in Fig. 4 comprises a conical pipe section 8 and the insert 2 is attached thereto. The pipe section 8 is sealed to an inner ring 30 surrounded by a locking ring 31, and the edge of an opening in the surrounding bladder 1 is fixed between the locking ring 31 and the inner ring 30. The pipe section 8 illustrated in Fig. 4 and the inner ring preferably consist of hard resin or metal.

### Claims

1. A squeeze bag for a manually operated resuscitator, said squeeze bag comprising an outer airtight and flexible bladder (1), having a gas outlet (4) and a gas inlet (3), a self-expanding foldable insert (2) provided within said bladder (1), the gas outlet (4) having inserted therein coupling means for coupling the bladder (1) to a valve system of the resuscitator and the gas inlet (3) having inserted therein a gas inlet valve (10), characterized in that the gas outlet (4) is sealed to a sealing ring (5, 6) which surrounds and fits tightly to the coupling means which is in the form of a pipe section (8), said coupling means being detachable from said sealing ring (5, 6) and being connected with the insert (2), and that the gas inlet (3) is such that the inlet valve (10) may be removed and that subsequent to which the insert (2) and the pipe section (8) attached thereto can be removed through said gas inlet (3) with the insert (2) in folded state.

2. A squeeze bag according to claim 1, characterized in that the pipe section (8) has a slightly conical exterior shape, the diameter decreasing in the direction towards its free end.

3. A squeeze bag according to claim 2, characterized in that the exterior surface of the pipe section (8) comprises an annular bead (29) having an outer diameter which is slightly higher than the internal diameter of the sealing ring (5, 6).

4. A squeeze bag according to claim 1, characterized in that the sealing ring is composed of an inner ring (6) and an outer locking ring (5), and that the edge of the outlet (4) of the bladder (1) is fixed between these two rings (5, 6).

5. A squeeze bag according to claim 4, characterized in that the sealing ring is composed of an inner rigid ring (6) and an outer elastic locking ring (5).

6. A squeeze bag according to claim 1, characterized in that the pipe section (8) is connected with the insert (2) by placing the stretched edge of a hole (7) in the insert wall in an annular groove (9) provided on the exterior surface of the pipe section (8).

7. A squeeze bag according to claim 1, characterized in that the gas inlet valve (10) comprises an internal annular flange (11) and a co-operating external compression nut (12) of such a shape that the edge of the gas inlet (3) can be fixed between the internal annular flange (11) and the compression nut (12).

8. A squeeze bag according to claim 7, characterized in that an anti-friction ring (14) is provided between the compression nut (12) and the edge of the gas inlet (3) of the bladder (1).

### Patentansprüche

1. Quetschbeutel für eine handbetätigtes Beatmungsgerät, wobei der Quetschbeutel aus einer äußeren luftdichten flexiblen Blase (1) mit einem Gasauslaß (4) und einem Gaseinlaß (3) und aus einem in dem Beutel (1) angeordneten selbsttätig expandierenden, faltbaren Einsatz (2), wobei in den Gasauslaß (4) eine Kupplungseinrichtung eingesetzt ist, um den Beutel (1) mit einem Ventilsystem des Beatmungsgerätes zu kuppeln, und wobei in den Gaseinlaß (3) ein Gaseinlaßventil (10) eingesetzt ist, dadurch gekennzeichnet, daß mit dem Gasauslaß (4) ein Dichtungsring (5, 6) gasdicht verbunden ist, der die in Form eines Rohrabschnittes (8) ausgebildete Kupplungseinrichtung eng umgreift, wobei die Kupplungseinrichtung von dem Dichtungsring (5, 6) ablösbar und mit dem Einsatz (2) verbunden ist, und daß der Gaseinlaß (3) so ausgebildet ist, daß das Einlaßventil (10) abnehmbar ist und daß nachfolgend der Einsatz (2) und der daran befestigte Rohrabschnitt (8) durch den Gaseinlaß (3) entnommen werden können, wobei sich der Einsatz (2) im zusammengefalteten Zustand befindet.

2. Quetschbeutel nach Anspruch 1, dadurch gekennzeichnet, daß der Rohrabschnitt (8) eine leicht konische Außenform hat, wobei der Durchmesser zum seinem freien Ende hin abnimmt.

3. Quetschbeutel nach Anspruch 2, dadurch gekennzeichnet, daß die Außenfläche des Rohrabschnittes (8) einen Ringwulst (29) aufweist, dessen Außendurchmesser geringfügig größer ist als der Innendurchmesser des Dichtringes (5, 6).

4. Quetschbeutel nach Anspruch 1, dadurch gekennzeichnet, daß der Dichtung aus einem inneren Ring (6) und aus einem äußeren Verriegelungsring (5) besteht und daß der Rand des Auslasses (4) des Beutels zwischen diesen beiden Ringen (5, 6) festgelegt ist.

5. Quetschbeutel nach Anspruch 4, dadurch gekennzeichnet, daß der Dichtring aus einem starren inneren Ring (6) und aus einem elastischen äußeren Verriegelungsring (5) besteht.

6. Quetschbeutel nach Anspruch 1, dadurch gekennzeichnet, daß der Rohrabschnitt (8) mit dem Einsatz (2) verbunden ist, in dem der gedehnte Rand einer in der Wand des Einsatzes ausgebildeten Öffnung (7) in einer an der Außenfläche des Rohrabschnitts (8) ausgebildeten Ringnut (9) angeordnet wird.

7. Quetschbeutel nach Anspruch 1, dadurch gekennzeichnet, daß das Gaseinlaßventil (10) einen inneren Ringflansch (11) und eine damit zusammenwirkende äußere Anpreßmutter (12) umfaßt, die eine solche Form hat, daß der Rand des Gaseinlasses (3) zwischen dem inneren Ringflansch (11) und der Anpreßmutter (12) eingespannt werden kann.

8. Quetschbeutel nach Anspruch 7, dadurch gekennzeichnet, daß ein Gleitring (14) zwischen der Anpreßmutter (12) und dem Rand das Gaseinlasses (3) des Beutels (1) angeordnet ist.

**Revendications**

1. Poche comprimable pour réanimateur commandé manuellement, ladite poche comprenant une vessie extérieure (1) souple et étanche munie d'une sortie de gaz (4) et d'une entrée de gaz (3), une garniture intérieure dépliable autodéployable (2) prévue à l'intérieur de ladite vessie, un moyen de raccordement logé dans la sortie de gaz (4) pour raccorder la vessie (1) à un réseau de valve du réanimateur, une valve d'entrée de gaz (10) logée dans l'entrée de gaz (3), caractérisée en ce que la sortie de gaz (4) est fixée à un anneau d'étanchéité (5, 6) qui entoure le, et s'adapte hermétiquement au moyen de raccordement qui présente la forme d'un tronçon de tuyau (8), ledit moyen de raccordement pouvant être séparé de l'anneau d'étanchéité (5, 6) et étant raccordé à la garniture intérieure (2) et en ce que l'entrée de gaz (3) est prévue de telle sorte que la valve d'entrée (10) puisse être retirée puis que la garniture intérieure (2) et le tronçon de tuyau (8) qui y est relié puissent être retirés par ladite entrée de gaz (3), la garniture intérieure (2) étant repliée.

2. Poche comprimable selon la revendication 1, caractérisée en ce que le tronçon de tuyau (8) a une forme extérieure légèrement conique, son diamètre diminuant en direction de son extrémité libre.

3. Poche comprimable selon la revendication 2, caractérisée en ce que la surface extérieure du tronçon de tuyau (8) comporte un bourrelet annulaire (39) dont le diamètre extérieur est légèrement supérieur au diamètre intérieur de l'anneau d'étanchéité (5, 6).

4. Poche comprimable selon la revendication 1, caractérisée en ce que l'anneau d'étanchéité est constitué d'un anneau intérieur (6) et d'un anneau extérieur de verrouillage (5) et en ce que le bord de l'orifice de sortie (4) de la vessie (1) est fixé entre ces deux anneaux (5, 6).

5. Poche comprimable selon la revendication 4, caractérisée en ce que l'anneau d'étanchéité est constitué d'un anneau intérieur rigide (6) et d'un anneau extérieur de verrouillage élastique (5).

6. Poche comprimable selon la revendication 1, caractérisée en ce que le tronçon de tuyau (8) est relié à la garniture intérieure (2) par placement du bord étiré d'un orifice (7) de la paroi de la garniture intérieure dans une gorge annulaire (9) prévue sur la surface extérieure du tronçon de tuyau (8).

7. Poche comprimable selon la revendication 1, caractérisée en ce que la valve d'entrée de gaz (10) comporte un talon annulaire intérieur (11) et un écrou de compression externe (12) associé de forme telle que le bord de l'orifice d'entrée de gaz (3) puisse être fixé entre le talon annulaire intérieur (11) et l'écrou de compression (12).

8. Poche comprimable selon la revendication 7, caractérisée en ce qu'un anneau anti-friction (14) est prévu entre l'écrou de compression (12) et le bord de l'orifice d'entrée de gaz (3) de la vessie (1).

0 143 208

FIG.1

FIG.2

FIG.4

FIG.3

0 143 208